# EUROPEAN PATENT APPLICATION

(11) **EP 2 130 823 A1**
(43) Date of publication of application: **09.12.2009**
(21) Application number: 08736738.9
(22) Date of filing: 14.03.2008
(51) Int. Cl.: C07D 239/95, C07D 239/96, A61K 31/517, A61K 31/519, A61P 29/00, A61P 37/00

(54) **COMPOUND THAT IS A DUAL INHIBITOR OF ENZYMES PDE7 AND/OR PDE4, PHARMACEUTICAL COMPOSITIONS AND USES THEREOF**

(30) Priority: 22.03.2007 ES 200700762
(71) Applicant: Consejo Superior De Investigaciones Científicas, 28006 Madrid (ES); Instituto de Salud Carlos III, 28029 Madrid (ES)
(72) Inventor: GIL AYUSO-GONTÁN,Carmen, E-28006 Madrid (ES); CASTAÑO CALDUCH, Tania, E-28006 Madrid (ES); CAMPILLO MARTÍN, Nuria, E-28006 Madrid (ES); BALLESTER JAREÑO, Sara, E-28029 Madrid (ES); GONZÁLEZ GARCÍA, Coral, E-28029 Madrid (ES); HERNÁNDEZ TORRES, Francisco, Javier, E-28029 Madrid (ES)
(74) Representative: Pons Ariño, Angel
(86) International application number: PCT/ES2008/070051
(87) International publication number: WO 2008/113881

(57) **Abstract**

The invention relates to a series of dual inhibitors of enzymes PDE7 and PDE4, having formula (I) and to the use thereof in the production of pharmaceutical compositions for the treatment of inflammatory and/or autoimmune processes.

## Description

### SECTOR OF THE TECHNIQUE

The invention is aimed at the field of medical chemistry, more specifically at dual inhibitors of enzymes PDE7 and PDE4 and their potential use in the preparation of pharmaceutical compositions for the treatment of inflammatory or autoimmune processes; and therefore, it relates to the pharmaceutical sector.

### STATE OF THE TECHNIQUE

The enzymatic activity of Phosphodiesterases (PDE) consists of degrading cyclic nucleotides (cAMP and cGMP) through hydrolytic breakage of the 3'-phosphodiester bond, giving rise to the corresponding inactive form of 5'-monophosphate (Conti, M.; Jin, S. L., The molecular biology of cyclic nucleotide phosphodiesterases. Prog. Nucleic Acid Res. Mol. Biol. 1999, 63, 1-38). cAMP and cGMP are generated through the action of adenylate cyclase and guanylate cyclase respectively, acting as secondary messengers in the transduction of intracellular signs. One way of increasing the intracellular levels of cAMP or cGMP is through the inhibition of PDEs, since they are their only route of degradation (Vig, M.; George, A.; Sen, R.; Durdik, J.; Rath, S.; Bal, V., Commitment of activated T cells to secondary responsiveness is enhanced by signals mediated by cAMP-dependent protein kinase A-I. Mol. Pharmacol. 2002, 62, 1471-1481; Ritcher, W., 3',5'-Cyclic nucleotide phosphodiesterases class III: Members, structure, and catalytic mechanism. Proteins 2002, 46, 278-286). The interest in developing specific PDE inhibitors is based on the antiinflammatory and immunosuppressor properties shown by agents capable of increasing the levels of intracellular cAMP (Essayan, D. M., Cyclic nucleotide phosphodiesterase (PDE) inhibitors and immunomodulation. Biochem. Pharmacol. 1999, 57, 965-973; Allison, A. C., Immunosuppressive drugs: The first 50 years and a glance forward. Immunopharmacology 2000, 47, 63-83). Therefore, selective inhibitors of cAMP-specific PDEs could be of interest as a therapy in the treatment of various diseases (Lugnier, C. Cyclic nucleotide phosphodiesterase (PDE) superfamily: A new target for the development of specific therapeutic agents. Pharmacol Ther. 2006, 109, 366-398), fundamentally alterations of the immune system, such as multiple sclerosis (Dyke, H. J.; Montana, J. G., Update on the therapeutic potential of PDE4 inhibitors. Expert Opin. Invest. Drugs 2002, 11, 1-13), inflammatory alterations (Spina, D., Phosphodiesterase-4 inhibitors in the treatment of inflammatory lung disease. Drugs 2003, 63, 2575-2594) and also disorders of the central nervous system, such as depression, cerebral ischemia damage, and Alzheimer's (Menniti, F. S.; Faraci, W. S.;Schmidt, C. J. Phosphodiesterases in the CNS: targets for drug development. Nat Rev Drug Discov. 2006, 5, 660-670).

Of the 11 described families of PDEs, those expressed in T-lymphocytes are mainly 3B, 4A, 4B, 4D and 7A1 (Ekholm, D.; Hemmer, B.; Gao, G.; Vergelli, M.; Martin, R.; Manganiello, V., Differential expression of cyclic nucleotide phosphodiesterase 3 and 4 activities in human T cell clones specific for myelin basic protein. J. Immunol. 1997, 159, 1520-1529; Erdogan, S.; Houslay, M. D., Challenge of human Jurkat T-cells with the adenylate cyclase activator forskolin elicits major changes in cAMP phosphodiesterase (PDE) expression by up-regulating PDE3 and inducing PDE4D1 and PDE4D2 splice variants as well as down-regulating a novel PDE4A splice variant. Biochem. J. 1997, 321, 165-175; Giembycz, M. A.; Corrigan, C. J.; Seybold, J.; Newton, R.; Barnes, P. J., Identification of cyclic AMP phosphodiesterases 3, 4 and 7 in human CD4+ and CD8+T-lymphocytes: Role in regulating proliferation and the biosynthesis of interleukin-2. Br. J. Pharmacol. 1996, 118, 1945-1958). Given that PDE4 is the major isoenzyme in T-cells, the development of PDE4 inhibitors has been the focus of most interest. In fact, selective inhibitors of this enzyme have demonstrated the capacity to reduce the production of inflammatory cytokines (Giembycz, M. A.; Corrigan, C. J.; Seybold, J.; Newton, R.; Barnes, P. J., Identification of cyclic AMP phosphodiesterases 3, 4 and 7 in human CD4+ and CD8+T-Iymphocytes: Role in regulating proliferation and the biosynthesis of interleukin-2. Br. J. Pharmacol. 1996, 118, 1945-1958; Manning, C. D.; Burman, M.; Christensen, S. B.; Cieslinski, L. B.; Essayan, D. M.; Grous, M.; Torphy, T. J.; Barnette, M. S., Suppression of human inflammatory cell function by subtype-selective PDE4 inhibitors correlates with inhibition of PDE4A and PDE4B. Br. J. Pharmacol. 1999, 128, 1393-1398), which may have an application in the treatment of asthma, rheumatoid arthritis, and multiple sclerosis among other diseases (Houslay, M. D.; Schafer, P.; Zhang, K. Y. J. Phosphodiesterase-4 as a therapeutic target. Drug Discov. Today 2005, 10, 1503-1519). Currently, two PDE4 inhibitors, cilomilast and roflumilast, are in clinical phase III for the treatment of two diseases characterised by an immune and inflammatory response, asthma and chronic obstructive lung disease (COLD) (Chung, K. F. Phosphodiesterase inhibitors in airways disease. Eur. J. Pharmacol. 2006, 533, 110-117). However, these inhibitors demonstrate a high toxicity due to their strong emetic nature. With a view to avoiding these negative effects, various strategies have been considered, including the development of a second generation of PDE4 inhibitors with an improved pharmacokinetic profile and fewer secondary effects (Giembycz, M. Life after PDE4: overcoming adverse events with dual-specificity phosphodiesterase inhibitors. Curr. Opin. Pharm. 2005, 5, 238-244).

Another alternative is the development of inhibitors against other cAMP-selective PDEs that are expressed in immune cells. Specifically, the inhibition of PDE7 could be an approach for the treatment of diseases dependent on T-cell activation, since the potential for inhibiting the activity of Th1 cells by blocking the activity of this enzyme has been demonstrated (Li, L.; Yee, C.; Beavo, J. A., CD3- and CD28-dependent induction of PDE7 required for T cell activation. Science 1999, 283, 848-851; Nakata, A.; Ogawa, K.; Sasaki, T.; Koyama, N.; Wada, K.; Kotera, J.; Kikkawa, H.; Omori, K.; Kaminuma, O. Potential role of phosphodiesterase 7 in human T cell function: comparative effects of two phosphodiesterase inhibitors. Clin. Ex.p Immunol. 2002, 128, 460-466). However, experiments based on the use of PDE7A^{-/-} *knock out* mice have not confirmed the role of PDE7A in the proliferation of T-cells, and suggest that this enzyme could fulfil a different function in the regulation of the humoral immune response (Yang, G.; Mclntyre, K. W.; Townsend, R. M.; Shen, H. H.; Pitts, W. J.; Dodd, J. H.; Nadler, S. G.; McKinnon, M.; Watson, A. J., Phosphodiesterase 7A-deficient mice have functional T cells. J Immunol. 2003, 171, 6414-20). Therefore, the use of PDE7-selective inhibitors is fundamental for elucidating the real potential of PDE7A as a pharmacological target for the treatment of immune response alterations (Castro, A.; Jerez, M. J.; Gil, C.; Martínez, A. Cyclic nucleotide phosphodiesterases and their role in immunomodulatory responses: Advances in the development of specific phosphodiesterase inhibitors Med. Res. Rev. 2005, 25, 229-244). In this context, derivatives of benzo- and benzothienothiadiazine developed by the inventors of the present patent were the first described heterocyclic inhibitors of PDE7 (Martínez, A.; Castro, A.; Gil, C.; Miralpeix, M.; Segarra, V.; Doménech, T.; Beleta, J.; Palacios, J.; Ryder, H.; Miró, X.; Bonet, C.; Casacuberta, J.; Azorín, F.; Piña, B.; Puigdoménech, P. Benzyl derivatives of 2,1,3-benzo- and benzothieno [3,2-a] thiadiazine 2,2-dioxides: First phosphodiesterase 7 inhibitors. J. Med. Chem., 2000, 43, 683-689; Castro, A.; Abasolo, M. I.; Gil, C.; Segarra, V.; Martínez, A. CoMFA of benzyl derivatives of 2,1,3-benzo and benzothieno [3,2-a] thiadiazine 2,2-dioxides: clues for the design of phosphodiesterase 7 inhibitors. Eur. J. Med. Chem. 2001, 36, 333-338). Since then, spirotricyclic derivatives, sulphonamides, purines, and pyrimidines among others, have been described as inhibitors of PDE7 (Vergne, F.; Bernardelli, P.; Chevalier, E. PDE7 Inhibitors: Chemistry and Potential Therapeutic Utilites. Ann. Rep. Med. Chem. 2005, 40, 227-241; Giembycz, M. A.; Smith, S. J. Phosphodiesterase 7 (PDE7) as a therapeutic target. Drugs Fut. 2006, 31, 207-229).

The development of PDE7-selective inhibitors has made it possible to study the function of PDE7A in different cells and tissues. However, although the inhibition of PDE7A does not reduce the proliferation of T-cells *per se,* it does considerably increase the effect that PDE4 inhibitors have on increasing the levels of cAMP (Smith, S. J.; Cieslinski, L. B.; Newton, R.; Donnelly, L. E.; Fenwick, P. S.; Nicholson, A. G.; Barnes, P. J.; Barnette, M. S.; Giembycz, M. A. Discovery of BRL 50481 [3-(N,N-dimethylsulfonamide)-4-methyl-nitrobenzene], a selective inhibitor of phosphodiesterase 7: in vitro studies in human monocytes, lung macrophages, and CD8+ T-Iymphocytes. Mol. Pharmacol. 2004, 66, 1679-89).

Consequently, the use of dual PDE4-PDE7 inhibitors, could exercise control over determined functions of Th lymphocytes that are involved in inflammation (Hatzelmann, A.; Marx, D.; Steinhilber, W.; Altana Pharma AG. 2002. Phthalazinone derivatives useful as PDE4/7 inhibitors (WO02085906); Pitts, W. J.; Watson, A. J.; Dodd, J. H.; Bristol Myers Squibb. 2002. Dual inhibitors of PDE7 and PDE4. World patent (WO 02088079)), comprising a good strategy in the control of inflammatory or autoimmune processes, for example in multiple sclerosis (MS), and would also be less restricted by the secondary effects.

### DESCRIPTION OF THE INVENTION

### Brief description

One object of this invention is a compound (understood as meaning a family of compounds) with a dual inhibitory activity on enzymes PDE7 and/or PDE4, hereinafter the compound of the present invention, which presents the formula (I): 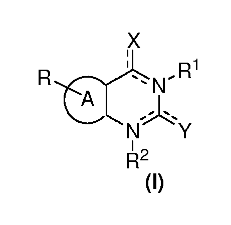 wherein:
A, is a carbocycle or optionally substituted fused heterocycle of 5, 6 or 7 members saturated or unsaturated,
--- may be a double bond;
X and Y, are independently selected from among the group consisting of hydrogen, alkyl, =O, =S, -N(alkyl), -N(aryl), aryl, O-alkyl, O-aryl, S-alkyl or -S-aryl; and
R, R¹ and R², are independently selected from among the group consisting of hydrogen, halogen, alkyl, haloalkyl, aryl, cycloalkyl, (Z)ₙ-aryl, heteroaryl, -OR³; -C(O)OR³, -(Z)ₙ-C(O)OR³ or -S(O)ₜ-,
or a pharmaceutically acceptable salt, derivative, prodrug, solvate or stereoisomer thereof.

Exception: when A is benzene without substituting, X=O,Y=S
when A is benzene without substituting, X=O,Y=O,
when A is benzene without substituting, X=O,Y=S-Me
when A is thiophene without substituting, X=O,Y=S, and
when A is benzothiophene without substituting, X=O,Y=S

Another additional object of the present invention is constituted by a pharmaceutical composition useful in the treatment of inflammatory and autoimmune diseases, hereinafter the pharmaceutical composition of the invention, which comprises a compound, in a therapeutically effective quantity, of formula (I), or mixtures thereof, a pharmaceutically acceptable salt, derivative, prodrug, solvate or stereoisomer thereof together with a pharmaceutically acceptable carrier, adjuvant or vehicle, for administration to a patient.

Another additional object of the present invention is constituted by the use of a compound of the invention of formula (I), or its mixtures for the preparation of a medicine, hereinafter use of a compound of the invention, directed at treating inflammatory and/or autoimmune pathologies or diseases, including, but not limited to, intestinal inflammatory disease, inflammatory joint pathologies, atopic dermatitis and other inflammatory skin pathologies, neuritis, encephalitis, encephalomyelitis and inflammatory pathologies that affect the central nervous system (multiple sclerosis) or peripheral nervous system, myositis, vasculitis, systemic lupus erythematosus, infectious diseases with inflammation symptoms, host rejection of grafts, conjunctivitis, and inflammatory pathologies of the eye, ear, and mucous membranes.

### Detailed description

The present invention is based on the fact that the inventors have demonstrated that compounds of formula (I) are dual inhibitors of enzymes PDE7 and/or PDE4, more specifically, of enzymes PDE7A1 and/or PDE4D2 (Example 5), meaning that they can be used in the preparation of pharmaceutical compounds for the treatment of inflammatory or autoimmune processes.

Therefore, one object of the present invention is a compound (understood to mean a family of compounds) with a dual inhibitor activity of the enzymes PDE7 and/or PDE4, hereinafter the compound of the present invention, which presents the formula (I): 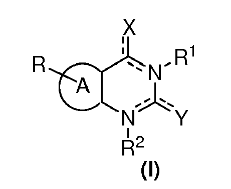 wherein:
A, is a carbocycle or optionally substituted fused heterocycle of 5, 6 or 7 members saturated or unsaturated,
--- may be a double bond;
X and Y, are selected independently from among the group consisting of hydrogen, alkyl, =O, =S, -N(alkyl), -N(aryl), aryl, O-alkyl, O-aryl, S-alkyl or -S-aryl; and
R, R¹ and R², are selected independently from among the group consisting of hydrogen, halogen, alkyl, haloalkyl, aryl, cycloalkyl, (Z)ₙ-aryl, heteroaryl, -OR³; -C(O)OR³, -(Z)ₙ-C(O)OR³ or -S(O)ₜ-,
or a pharmaceutically acceptable salt, derivative, prodrug, solvate or stereoisomer thereof.

Exception: when A is benzene without substituting, X=O,Y=S
when A is benzene without substituting, X=O,Y=O,
when A is benzene without substituting, X=O,Y=S-Me
when A is thiophene without substituting, X=O,Y=S, and
when A is benzothiophene without substituting, X=O,Y=S

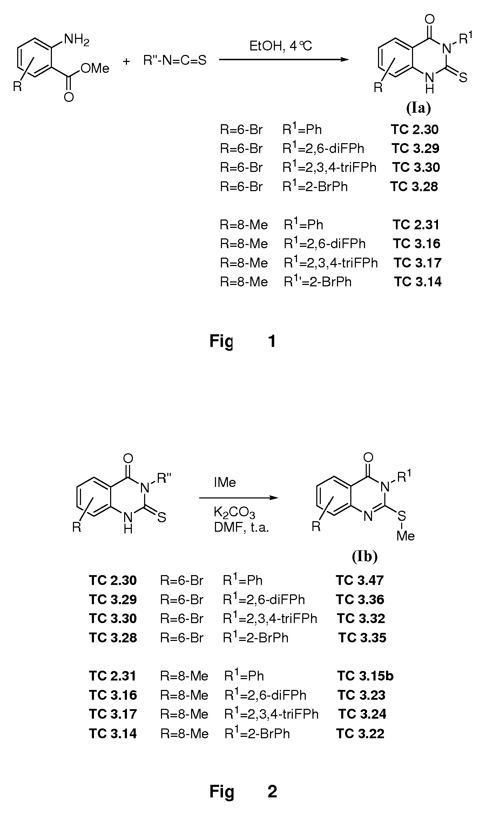

A particular object of the invention is constituted by a compound of the invention with the formula 4-oxo-2-thioxo-1,2,3,4-tetrahydroquinazoline (Compound la, Figure 1), and more preferably, belonging, by way of illustration and without limiting the scope of the invention, to the following group:
- 6-Bromo-3-phenyl-4-oxo-2-thioxo-1,2,3,4-tetrahydroquinazoline (TC.2.30),
- 6-Bromo-3-(2,6-difluorophenyl)-4-oxo-2-thioxo-1,2,3,4-tetrahydroquinazoline (TC.3.29),
- 6-Bromo-3-(2,3,4-trifluorophenyl)-4-oxo-2-thioxo-1,2,3,4-tetrahydroquinazoline (TC.3.30),
- 6-Bromo-3-(2-bromophenyl)-4-oxo-2-thioxo-1,2,3,4-tetrahydroquinazoline (TC.3.28),
- 3-(2,6-Difluorophenyl)-8-methyl--4-oxo-2-thioxo-1,2,3,4-tetrahydroquinazoline (TC.3.16),
- 3-(2,3,4-Trifluorophenyl)-8-methyl-4-oxo-2-thioxo-1,2,3,4-tetrahydroquinazoline (TC.3.17), and
- 3-(2-Bromophenyl)-8-methyl-4-oxo-2-thioxo-1,2,3,4-tetrahydroquinazoline (TC.3.14).

Another particular object of the invention is constituted by a compound of the invention with the formula 2-methylthio-4-oxo-3,4-dihydroquinazoline (Compound Ib, Figure 2), and more preferably, belonging, by way of illustration and without limiting the scope of the invention, to the following group:
- 6-Bromo-3-phenyl-2-methylthio-4-oxo-3,4-dihydroquinazoline(TC.3.47),
- 6-Bromo-3-(2,6-difluorophenyl)-2-methylthio-4-oxo-3,4-dihydroquinazoline(TC.3.36),
- 6-Bromo-3-(2,3,4-trifluorophenyl)-2-methylthio-4-oxo-3,4-dihydroquinazoline(TC.3.32),
- 6-Bromo-3-(2-bromophenyl)-2-methylthio-4-oxo-3,4-dihydroquinazoline(TC.3.35),
- 3-Phenyl-8-methyl-2-methylthio-4-oxo-3,4-dihydroquinazoline(TC.3.15b),
- 3-(2,6-Difluorophenyl)-8-methyl-2-methylthio-4-oxo-3,4-dihydroquinazoline(TC.3.23),
- 3-(2,3,4-Trifluorophenyl)-8-methyl-2-methylthio-4-oxo-3,4-dihydroquinazoline(TC.3.24), and
- 3-(2-Bromophenyl)-8-methyl-2-methylthio-4-oxo-3,4-dihydroquinazoline(TC.3.22).

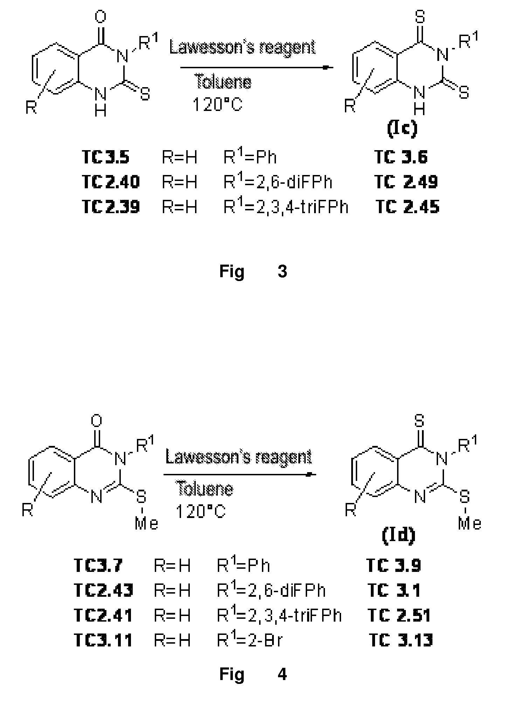

Another particular object of the invention is constituted by a compound of the invention with the formula 2,4-dithioxo-1,2,3,4-tetrahydroquinazoline (Compound Ic, Figure 3), and more preferably, belonging, by way of illustration and without limiting the scope of the invention, to the following group:
- 3-Phenyl-2,4-dithioxo-1,2,3,4-tetrahydroquinazoline (TC.3.6),
- 3-(2,6-Difluorophenyl)-2,4-dithioxo-1,2,3,4-tetrahydroquinazoline (TC.2.49), and
- 3-(2,3,4-Trifluorophenyl)-2,4-dithioxo-1,2,3,4-tetrahydroquinazoline (TC.2.45).

Another particular object of the invention is constituted by a compound of the invention with the formula 2-methylthio-4-thioxo-3,4-dihydroquinazoline (Compound Id, Figure 4), and more preferably, belonging, by way of illustration and without limiting the scope of the invention, to the following group:
- 3-Phenyl-2-methylthio-4-thioxo-3,4-dihydroquinazoline(TC.3.9),
- 3-(2,6-Difluorophenyl)-2-methylthio-4-thioxo-3,4-dihydroquinazoline(TC.3.1),
- 3-(2,3,4-Trifluorophenyl)-2-methylthio-4-thioxo-3,4-dihydroquinazoline(TC.2.51), and
- 3-(2-Bromophenyl)-2-methylthio-4-thioxo-3,4-dihydroquinazoline(TC.3.13).

The compounds of the present invention represented by the formula (I), and more specifically, the compounds of formula Ia, Ib, Ic and Id, and the specific compounds belonging to these sub-families as described above may include isomers, depending on the presence of multiple bonds (for example, Z, E), including optical or enantiomeric isomers, depending on the presence of chiral centres. The isomers, enantiomers, or individual diastereoisomers and mixtures thereof fall within the scope of the present invention. The enantiomers or individual diastereoisomers, as well as the mixtures thereof, may be separated using conventional techniques.

As referred to herein, the term "derivative" includes pharmaceutically acceptable compounds, in other words, derivatives of the compounds of the formula (I) which may be used in the preparation of a medicine, as well as pharmaceutically unacceptable derivatives since these may be useful in the preparation of pharmaceutically acceptable derivatives. The nature of the pharmaceutically acceptable derivative is not critical as long as it is pharmaceutically acceptable.

At the same time, the scope of the present invention includes the prodrugs of the compounds of formula (I). The term "prodrug" as referred to herein includes any compound derived from a compound of formula (I), for example, esters, including the esters of carboxylic acids, the esters of amino acids, phosphate esters, sulphonate esters of metal salts, etc., carbamates, amides, etc., which, when administered to an individual is capable of providing, directly or indirectly, said compound of formula (I) in said individual. Advantageously, such derivative is a compound that increases the bioavailability of the compound of formula (I) when administered to an individual or liberates the compound of formula (I) in a biological compartment. The nature of such derivative is not critical as long as it can be administered to an individual and provides the compound of formula (I) in a biological compartment of an individual. The preparation of such prodrug can be effected through conventional methods known to experts in the field.

The compounds of the invention may be in crystalline form as free compounds or as solvates and the intention is for both forms to be within the scope of the present invention. In this regard, the term "solvate", as used herein, includes both pharmaceutically acceptable solvates, in other words, solvates of the compound of formula (I) that may be used in the preparation of a medicine, as well as pharmaceutically unacceptable solvates, which may be useful in the preparation of pharmaceutically acceptable solvates or salts. The nature of the pharmaceutically acceptable solvate is not critical as long as it is pharmaceutically acceptable. In a particular embodiment, the solvate is a hydrate. The solvates may be obtained through conventional solvation methods well known to technicians in the field.

For their application in therapy, the compounds of formula (I), their isomers, salts, prodrugs or solvates, will be, preferably, in a pharmaceutically acceptable or substantially pure form, in other words, will have a pharmaceutically acceptable level of purity excluding standard pharmaceutical additives such as solvents and carriers, and not including material considered toxic at normal dosage levels. The purity levels for the active principle are preferably more than 50%, more preferably more than 70%, more preferably more than 90%. In a preferred embodiment, they are more than 95% of the compound of formula (I), or of its salts, solvates or prodrugs.

Unless otherwise indicated, the compounds of the invention also include compounds that differ only in the presence of one or more isotopically enriched atoms. For example, compounds with said structure, excluding the substitution of one hydrogen by one deuterium or by tritium, or the substitution of one carbon for one carbon enriched in ¹³C or ¹⁴C or one nitrogen enriched in ¹⁵N, are within the scope of this invention.

The compounds described in the present invention, their pharmaceutically acceptable salts, prodrugs and/or solvates as well as the pharmaceutical compositions containing them may be used in conjunction with other additional drugs in order to offer combination therapy. Said additional drugs may form part of the same pharmaceutical composition, or, alternatively, may be provided in the form of a separate composition for simultaneous or non-simultaneous administration with the pharmaceutical composition comprising a compound of formula (I), or prodrug, solvate, derivative or pharmaceutically acceptable salt thereof.

The compounds of the present invention of formula (I) may be obtained or produced via chemical synthesis or obtained from a natural matter of different origin. In the present invention a synthetic route is described for the compounds of the invention of formula (I) and their families (see Figure 1 to 4, and Examples 1 to 4), which may be carried out in practice using the data described in the present invention by an expert technician in the field. The synthesis procedures for the compounds of the invention Ia, Ib, Ic and Id, as described in the invention, constitute another object of the present invention.

Another additional object of the present invention is constituted by a pharmaceutical composition useful for the treatment of inflammatory and autoimmune diseases, hereinafter pharmaceutical composition of the invention, which comprises a compound, in a therapeutically effective quantity, of formula (I), or mixtures thereof, a pharmaceutically acceptable salt, derivative, prodrug, solvate or stereoisomer thereof, together with a pharmaceutically acceptable carrier, adjuvant or vehicle, for administration to a patient.

Another particular object of the invention is constituted by the pharmaceutical composition of the invention wherein the compound of formula (I) belongs to one, or to several, of the following families of compounds:
- 4-oxo-2-thioxo-1,2,3,4-tetrahydroquinazoline (Compound Ia, Figure 1),
- 2-methylthio-4-oxo-3,4-dihydroquinazoline (Compound Ib, Figure 2),
- 2,4-dithioxo-1,2,3,4-tetrahydroquinazoline (Compound Ic, Figure 3), and
- 2-methylthio-4-thioxo-3,4-dihydroquinazoline (Compound Id, Figure 4),
and, more preferably, a compound belonging, by way of illustration and without limiting the scope of the invention, to the following group:
- 6-Bromo-3-phenyl-4-oxo-2-thioxo-1,2,3,4-tetrahydroquinazoline (TC.2.30),
- 6-Bromo-3-(2,6-difluorophenyl)-4-oxo-2-thioxo-1,2,3,4-tetrahydroquinazoline (TC.3.29),
- 6-Bromo-3-(2,3,4-trifluorophenyl)-4-oxo-2-thioxo-1,2,3,4-tetrahydroquinazoline (TC.3.30),
- 6-Bromo-3-(2-bromophenyl)-4-oxo-2-thioxo-1,2,3,4-tetrahydroquinazoline (TC.3.28),
- 3-(2,6-Difluorophenyl)-8-methyl-4-oxo-2-thioxo-1,2,3,4-tetrahydroquinazoline (TC.3.16),
- 3-(2,3,4-Trifluorophenyl)-8-methyl-4-oxo-2-thioxo-1,2,3,4-tetrahydroquinazoline (TC.3.17),
- 3-(2-Bromophenyl)-8-methyl-4-oxo-2-thioxo-1,2,3,4-tetrahydroquinazoline (TC.3.14),
- 6-Bromo-3-phenyl-2-methylthio-4-oxo-3,4-dihydroquinazoline(TC.3.47),
- 6-Bromo-3-(2,6-difluorophenyl)-2-methylthio-4-oxo-3,4-dihydroquinazoline(TC.3.36),
- 6-Bromo-3-(2,3,4-trifluorophenyl)-2-methylthio-4-oxo-3,4-dihydroquinazoline(TC.3.32),
- 6-Bromo-3-(2-bromophenyl)-2-methylthio-4-oxo-3,4-dihydroquinazoline(TC.3.35),
- 3-Phenyl-8-methyl-2-methylthio-4-oxo-3,4-dihydroquinazoline(TC.3.15b),
- 3-(2,6-Difluorophenyl)-8-methyl-2-methylthio-4-oxo-3,4-dihydroquinazoline(TC.3.23),
- 3-(2,3,4-Trifluorophenyl)-8-methyl-2-methylthio-4-oxo-3,4-dihydroquinazoline(TC.3.24),
- 3-(2-Bromophenyl)-8-methyl-2-methylthio-4-oxo-3,4-dihydroquinazoline(TC.3.22),
- 3-Phenyl-2,4-dithioxo-1,2,3,4-tetrahydroquinazoline (TC.3.6),
- 3-(2,6-Difluorophenyl)-2,4-dithioxo-1,2,3,4-tetrahydroquinazoline (TC.2.49),
- 3-(2,3,4-Trifluorophenyl)-2,4-dithioxo-1,2,3,4-tetrahydroquinazoline (TC.2.45).
- 3-Phenyl-2-methylthio-4-thioxo-3,4-dihydroquinazoline(TC.3.9),
- 3-(2,6-difluorophenyl)-2-methylthio-4-thioxo-3,4-dihydroquinazoline(TC.3.1),
- 3-(2,3,4-trifluorophenyl)-2-methylthio-4-thioxo-3,4-dihydroquinazoline(TC.2.51),
- 3-(2-bromophenyl)-2-methylthio-4-thioxo-3,4-dihydroquinazoline(TC.3.13),
or any of their R, S enantiomeric forms and/or their racemic mixtures.

The pharmaceutically acceptable adjuvants and vehicles that may be used in said compositions are the adjuvants and vehicles known to technicians in the field and commonly used in the preparation of therapeutic compositions.

In the sense used in this description, the expression "therapeutically effective quantity" refers to the quantity of the agent or compound capable of developing inhibition of the enzymes PDE7 and PDE4, calculated to produce the required effect and, in general, will be determined, among other causes, by the inherent characteristics of the compounds, as well as age, state of the patient, severity of the alteration or disorder, including route and frequency of administration.

In another particular embodiment, said therapeutic composition is prepared in solid form or in aqueous suspension, in a pharmaceutically acceptable solvent. The therapeutic composition provided by this invention may be administered via any appropriate route of administration, and to this effect said composition will be formulated in the suitable pharmaceutical format for the selected administration route. In a particular embodiment, the therapeutic composition provided by this invention will be administered by oral, topical, rectal or parenteral route (including subcutaneous, intraperitoneal, intradermic, intramuscular, intravenous, etc.). A review of the various pharmaceutical way of administering medicines and the excipients required to obtain them can be found, for example, in the "Treaty of Galenic Pharmacy" "Tratado de Farmacia Galénica", C. Faulí i Trillo, 1993, Luzán 5, S.A. Ediciones, Madrid, or in other usual or similar ones of the Spanish and US Pharmacopoeia.

Another additional object of the present invention is constituted by the use of a compound of the invention of formula (I), or mixtures thereof for the preparation of a medicine, hereinafter use of a compound of the invention, aimed at treating inflammatory and/or autoimmune diseases or pathologies, including, but not limited to, intestinal inflammatory disease, inflammatory joint pathologies, atopic dermatitis and other inflammatory skin pathologies, neuritis, encephalitis, encephalomyelitis and inflammatory pathologies affecting the central nervous system (multiple sclerosis) or peripheral nervous system, myositis, vasculitis, systemic lupus erythematosus, infectious diseases with inflammation systems, reactions of host rejection of grafts, conjunctivitis and inflammatory pathologies of the eye, ear, and mucous membranes.

The use of the compounds of the invention is compatible with their use in protocols wherein the compounds of formula (I), or mixtures thereof are used by themselves or in combination with other treatment or any other medical procedure.

### DESCRIPTION OF THE DRAWINGS

**Figure 1****.-** Synthesis diagram of the compounds of formula la of the invention.
**Figure 2****.-** Synthesis diagram of the compounds of formula Ib of the invention.
**Figure 3****.-** Synthesis diagram of the compounds of formula Ic of the invention.
**Figure 4****.-** Synthesis diagram of the compounds of formula Id of the invention.
**Figure 5****.** Measurement of the increase in intracellular cAMP of the derivatives TC 3.6 and TC 3.14.

### EXAMPLES OF THE INVENTION

### Example 1.- General procedure for obtaining the derivatives of 4-oxo-2-thioxo-1,2,3,4-tetrahydroquinazoline (Compounds la, Figure 1)

To a solution of the derivative of 2-aminobenzoic acid in corresponding ethanol the isothiocyanate corresponding in each case (1 equivalent) is added at 0ºC. The mixture of the reaction is agitated at 4ºC, and the formation of a precipitate is observed. The solid is isolated through vacuum filtration obtaining the required product. Only in the indicated cases was purification of the solid through column chromatography necessary.
- 6-Bromo-3-phenyl-4-oxo-2-thioxo-1,2,3,4-tetrahydroquinazoline (TC.2.30):
   Obtained following the general methodology described above. Reagents: 5-bromo-2-aminobenzoic acid (800 mg, 3.70 mmol), phenylisothiocyanate (0.44 mL, 3.70 mmol), ethanol (37.03 mL). Reaction time: 3 days. Product: white solid (493.70 mg, 40%).
   ¹H-RMN (300 MHz, DMSO-*d*₆), δ: 13.22 (sa, 1H, NH); 8.09 (d, 1H, *J*= 2.1 Hz, H-5); 8.03 (dd, 1H, *J*= 8.7 and 2.1 Hz, H-7); 7.50-7.37 (m, 4H, H-c, H-c', H-d, H-8); 7.35 (d, 2H, *J*= 7.3 Hz, H-b, H-b').
   ¹³C-RMN (75 MHz, DMSO-*d*₆), δ: 176.0 (C-2); 158.7 (C-4); 139.1 (C-a); 138.8 (C-8a); 138.1 (C-7); 129.2 (C-5); 128.9 (2C, C-c, C-c'); 128.8 (2C, C-b, C-b'); 128.1 (C-d); 118.2 (C-8); 118.0 (C-4a); 115.7 (C-6).
   EM (ES): m/z= 333, 335 (M + H)⁺, 355,
   357 (M + Na)⁺. 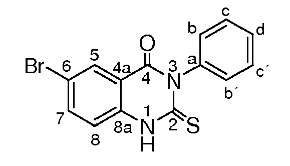
   Element analysis for C₁₄H₉BrN₂OS:
   Calculated: C% 50.46 H% 2.72 N% 8.41 S% 9.62.
   Found: C% 50.19 H% 2.88 N% 8.35 S% 9.34.
- 6-Bromo-3-(2,6-difluorophenyl)-4-oxo-2-thioxo-1,2,3,4-tetrahydroquinazoline (TC.3.29):
   Obtained following the general methodology described above. Reagents: 5-bromo-2-aminobenzoic acid (600 mg, 2.77 mmol), 2,6-difluorophenylisothiocyanate (0.35 mL, 2.77 mmol), ethanol (27.70 mL). Reaction time: 17 days. Product: white solid (354.30 mg, 35%).
   ¹H-RMN (300 MHz, DMSO-*d*₆), δ: 13.56 (sa, 1H, NH); 8.07 (d, 1H, *J*= 2.2 Hz, H-5); 8.01 (dd, 1H, *J*= 8.7 and 2.2 Hz, H-7); 7.66-7.56 (m, 1H, H-d); 7.42 (d, 1H, *J*= 8.7 Hz, H-8); 7.33-7.28 (m, 2H, Hc, Hc').
   ¹³C-RMN (75 MHz, DMSO-*d*₆), δ: 174.5 (C-2); 157.7 (dd, 2C, *J*= 248.7 and 4.4 Hz, C-b, C-b'); 157.6 (C-4); 139.2 (C-7); 138.9 (C-8a); 131.6 (t, 1C, *J*= 10.0 Hz, C-d); 129.4 (C-5); 118.6 (C-8); 116.7 (C-6); 116.5 (C-4a); 114.9 (t, 1C, *J*= 16.8 Hz, C-a); 112.2 (dd, 2C, *J*= 19.6 and 2,6 Hz, C-c, C-c').
   EM (ES): m/z= 369, 371 (M + H)⁺.
   Element analysis for C₁₄H₇BrF₂N₂OS:
   Calculated: C% 45.55 H% 1.91 N% 7.59 S% 8.69.
   Found: C% 45.31 H% 2.19 N% 7.47 S% 8.41.
   HPLC: purity = 98%.
- 6-Bromo-3-(2,3,4-trifluorophenyl)-4-oxo-2-thioxo-1,2,3,4-tetrahydroquinazoline (TC.3.30):
   Obtained following the general methodology described above. Reagents: 5-bromo-2-aminobenzoic acid (600 mg, 2.77 mmol), 1,3,4-trifluorophenylisothiocyanate (0.36 mL, 2.77 mmol), ethanol (27.70 mL). Reaction time: 4 days. Product: white solid (540.10 mg, 50%).
   ¹H-RMN (300 MHz, DMSO-*d*₆), δ: 13.43 (sa, 1H, NH); 8.04 (d, 1H, *J*= 2.2 Hz, H-5); 7.99 (dd, 1H, *J*= 8.7 and 2.2 Hz, H-7); 7.56-7.42 (m, 2H, H-c, H-b); 7.39 (d, 1H, *J*= 8.7 Hz, H-8).
   ¹³C-RMN (75 MHz, DMSO-*d*₆), δ: 175.1 (C-2); 158.2 (C-4); 150.3 (d, 1C, *J*= 257.1 Hz, C-d); 146.5 (dd, 1C, *J*= 246.4 and 14.4 Hz, C-b'); 139.2 (ddd, 1C, *J*= 231.2, 15.9 and 14.1 Hz, C-c'); 138.8 (C-8a); 138.7 (C-7); 129.3 (C-5); 126.0 (dd, 1C, *J*= 8.4 and 3.6 Hz, C-b); 123.8 (dd, 1C, *J*=10.7 and 4.1 Hz, C-a); 118.4 (C-8); 117.3 (C-4a); 116.4 (C-6); 112.7 (dd, 1C, *J*= 18.0 and 3.4 Hz, C-c).
   EM (IE): m/z= 366, 368 (M⁺-F, 91,100), 386, 388 (M⁺, 18, 19). 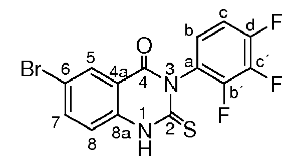
   Element analysis for C₁₄H₆BrF₃N₂OS:
   Calculated: C% 43.43 H% 1.56 N% 7.24 S% 8.28.
   Found: C% 43.11 H% 1.68 N% 7.07 S% 7.98.
- 6-Bromo-3-(2-bromophenyl)-4-oxo-2-thioxo-1,2,3,4-tetrahydroquinazoline (TC.3.28):
   Obtained following the general methodology described above. Reagents: 5-bromo-2-aminobenzoic acid (600 mg, 2.77 mmol), 2-bromophenylisothiocyanate (0.37 mL, 2.77 mmol), ethanol (27.70 mL). Reaction time: 9 days. Product: white solid (584.70 mg, 51 %).
   ¹H-RMN (300 MHz, DMSO-*d*₆), δ: 13.30 (sa, 1H, NH); 8.05 (d, 1H, *J*= 2.2 Hz, H-5); 7.98 (dd, 1H, *J*= 8.7 and 2.2 Hz, H-7); 7.76 (d, 1H, *J*= 7.5 Hz, H-c') 7.54-7.46 (m, 2H, H-c, H-b); 7.42-7.35 (m, 2H, H-8, H-d).
   ¹³C-RMN (75 MHz, DMSO-*d*₆), δ: 175.0 (C-2); 158.0 (C-4); 138.8 (C-7); 138.7 (C-8a); 138.2 (C-a); 132.9 (C-c'); 131.2 (C-b); 130.4 (C-d); 129.4 (C-5); 128.7 (C-c); 122.2 (C-b'); 118.3 (C-8); 117.5 (C-4a); 116.3 (C-6).
   EM (IE): m/z= 331, 333 (M⁺- Br, 100, 98).
   EM (ES): m/z= 413 (M + H)⁺, 825 (2M + H)⁺, 887 (2M + Na + K + H)⁺. 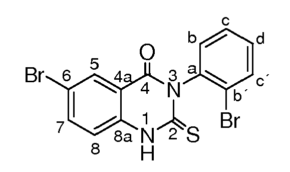
   Element analysis for C₁₄H₈Br₂N₂OS:
   Calculated: C% 40.80 H% 1.96 N% 6.80 S% 7.78.
   Found: C% 40.46 H% 2.23 N% 6.77 S% 7.65.
   HPLC: purity = 97%.
- 8-Methyl-3-phenyl-2-mercapto-4-oxo-3,4-dihydroquinazoline(TC.2.31): Obtained following the general methodology described above. Reagents: 3-methyl-2-aminobenzoic acid (50 mg, 0.33 mmol), phenylisothiocyanate (39.57 µL, 0.33 mmol), ethanol (3.30 mL). Reaction time: 3 days. The solid obtained was used in the following step without subsequent purification.
- 3-(2,6-Difluorophenyl)-8-methyl-4-oxo-2-thioxo-1,2,3,4-tetrahydroquinazoline (TC.3.16):
   Obtained following the general methodology described above. Reagents: 3-methyl-2-aminobenzoic acid (700 mg, 4.63 mmol), 2,6-difluorophenylisothiocyanate (0.59 mL, 4.63 mmol), ethanol (46.30 mL). Reaction time: 13 days. Purification: column chromatography using as eluent hexane/ethyl acetate (5:1). Product: white solid (642.70 mg, 46%).
   ¹H-RMN (300 MHz, CDCl₃), δ: 9.38 (sa, 1H, NH); 8.07 (d, 1H, *J*= 7.9 Hz, H-5); 7.59 (d, 1H, *J*= 7.3 Hz, H-7); 7.54-7.44 (m, 1H, H-d); 7.29 (dd, 1H, *J*= 7.9 and 7.3 Hz, H-6); 7.14-7.09 (m, 2H, Hc, Hc'); 2.50 (s, 3H, Me).
   ¹³C-RMN (75 MHz, CDCl₃), δ: 175.5 (C-2); 158.9 (C-4); 158.4 (dd, 2C, *J*= 251.4 and 4.2 Hz, C-b, C-b'); 137.3 (C-8a); 137.0 (C-7); 130.8 (t, 1C, *J*= 9.75 Hz, C-d); 126.9 (C-5); 124.7 (C-6); 122.5 (C-8); 115.5 (2C, C-a, C-4a); 112.0 (dd, 2C, *J*= 19.8 and 3.1 Hz, C-c, C-c'); 16.2 (Me).
   EM (ES): m/z= 305 (M + H)⁺, 327 (M + Na)⁺, 631 (2M + Na)⁺.
   EM (IE): m/z= 285 (M⁺-F, 100), 304 (M⁺, 60). 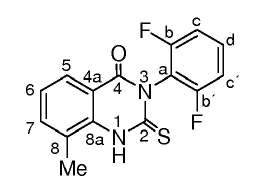
   Element analysis for C₁₅H₁₀F₂N₂OS:
   Calculated: C% 59.20 H% 3.31 N% 9.21 S% 10.54.
   Found: C% 59.10 H% 3.45 N% 9.40 S% 10.65.
- 3-(2,3,4-Trifluorophenyl)-8-methyl-4-oxo-2-thioxo-1,2,3,4-tetrahydroquinazoline (TC.3.17):
   Obtained following the general methodology described above. Reagents: 3-methyl-2-aminobenzoic acid (700 mg, 4.63 mmol), 2,3,4-trifluorophenylisothiocyanate (0.61 mL, 4.63 mmol), ethanol (46.30 mL). Reaction time: 4 days. Purification: column chromatography using as eluent hexane/ethyl acetate (3:1). Product: white solid (505.40 mg, 34%).
   ¹H-RMN (300 MHz, CDCl₃), δ: 9.36 (sa, 1H, NH); 8.01 (d, 1H, *J*= 7.8 Hz, H-5); 7.56 (dd, 1H, *J*= 7.4 and 0.5 Hz, H-7); 7.26 (dd, 1H, *J*= 7.8 and 7.4 Hz, H-6); 7.16-7.04 (m, 2H, H-c, H-b); 2.46 (s, 3H, Me).
   ¹³C-RMN (75 MHz, CDCl₃), δ: 175.8 (C-2); 159.4 (C-4); 151.6 (ddd, 1C, *J*= 251.0, 10.0 and 2.9 Hz, C-d); 147.6 (ddd, 1C, *J*= 253.1, 11.0 and 3.9 Hz, C-b'); 140.8 (ddd, 1C, *J*= 251.7, 15.8 and 13.8 Hz, C-c'); 137.2 (C-8a); 137.1 (C-7); 126.9 (C-5); 124.9 (C-6); 124.2 (dd, 1C, *J*= 8.1 and 4.1 Hz, C-b); 123.4 (dd, 1C, *J*= 10.9 and 4.4 Hz, C-a); 122.5 (C-8); 115.7 (C-4a); 112.1 (dd, 1C, *J*= 18.6 and 3.9 Hz, C-c); 16.2 (Me).
   EM (IE): m/z= 303 (M⁺-F, 100), 322 (M⁺, 61). 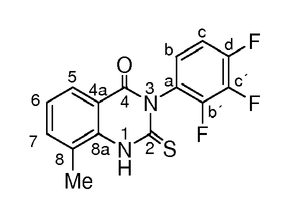
   Element analysis for C₁₅H₉F₃N₂OS:
   Calculated: C% 55.90 H% 2.81 N% 8.69 S% 9.95.
   Found: C% 55.84 H% 3.01 N% 8.57 S% 9.97.
   HPLC: purity = 99%.
- 3-(2-Bromophenyl)-8-methyl-4-oxo-2-thioxo-1,2,3,4-tetrahydroquinazoline (TC.3.14):
   Obtained following the general methodology described above. Reagents: 3-methyl-2-aminobenzoic acid (700 mg, 4.63 mmol), 2-bromophenylisothiocyanate (0.62 mL, 4.63 mmol), ethanol (46.30 mL). Reaction time: 5 days. Purification: column chromatography using as eluent hexane/ethyl acetate (8:1). Product: white solid (854.30 mg, 53%).
   ¹H-RMN (200 MHz, DMSO-*d*₆), δ: 11.98 (sa, 1H, NH); 7.86 (d, 1H, *J*= 7.9 Hz, H-5); 7.76 (d, 1H, *J*= 8.0 Hz, H-c'); 7.65 (d, 1H, *J*= 7.5 Hz, H-7); 7.56-7.46 (m, 2H, H-c, H-b); 7.42-7.35 (m, 1H, H-d); 7.29 (dd, 1 H, *J*= 7.9 and 7.5 Hz, H-6); 2.53 (s, 1H, Me).
   ¹³C-RMN (75 MHz, DMSO-*d*₆), δ: 175.4 (C-2); 159.0 (C-4); 138.3 (C-8a); 138.0 (C-a); 137.2 (C-7); 132.7 (C-c'); 131.1 (C-b); 130.2 (C-d); 128.6 (C-c); 125.3 (C-5); 124.7 (C-8); 124.4 (C-6); 122.2 (C-b'); 116.0 (C-4a); 17.4 (Me).
   EM (IE): m/z= 267 (M⁺- Br, 100). 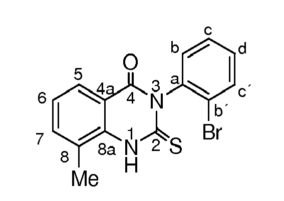
   Element analysis for C₁₅H₁₁BrN₂OS:
   Calculated: C% 51.89 H% 3.19 N% 8.07 S% 9.23.
   Found: C% 52.03 H% 3.32 N% 8.16 S% 9.16.
   HPLC: purity > 99%.

### Example 2.- General procedure for obtaining the derivatives of 2-methylthio-4-oxo-3,4-dihydroquinazoline (Compounds Ib, Figure 2).

To a solution of the derivative of 4-oxo-2-thioxo-1,2,3,4-tetrahydroquinazoline in corresponding DMF (1 equivalent) K₂CO₃ is added (1 equivalent) and agitated at room temperature during one hour. After this time, methyl iodide is added (1.5 equivalents) and agitated at room temperature. Next, the solvent is eliminated through evaporation at reduced pressure and the solid obtained is purified as indicated in each case.
- 6-Bromo-3-phenyl-2-methylthio-4-oxo-3,4-dihydroquinazoline(TC.3.47):
   Obtained following the general methodology described above. Reagents: **TC.2.30** (60 mg, 0.18 mmol), K₂CO₃ (24.90 mg, 0.18 mmol), DMF (3.91 mL), methyl iodide (16.81 µL, 0.27 mmol). Reaction time: 3 hours. Purification: column chromatography using as eluent hexane/ethyl acetate (4:1). Product: white solid (60.80 mg, 97%).
   ¹H-RMN (300 MHz, CDCl₃), δ: 8.35 (d, 1H, *J*= 2.2 Hz, H-5); 7.80 (dd, 1H, J= 8.6 and 2.2 Hz, H-7); 7.57-7.54 (m, 3H, H-c, H-c', H-d); 7.51 (d, 2H, J= 8.6 Hz, H-8); 7.33-7.27 (m, 2H, H-b, H-b'); 2.51 (Me).
   ¹³C-RMN (75 MHz, CDCl₃), δ: 160.7 (C-4); 158.8 (C-2); 146.8 (C-8a); 137.7 (C-7); 135.7 (C-a); 130.9 (C-d); 129.8 (2C, C-c, C-c'); 129.7 (C-5); 129.1 (2C, C-b, C-b'); 128.1 (C-8); 121.3 (C-4a); 119.0 (C-6); 15.5 (Me).
   EM (ES): m/z= 347, 349 (M + H)⁺, 717 (M + Na)⁺. 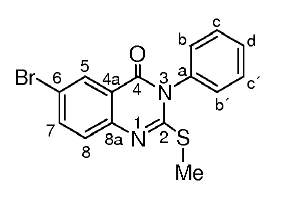
   Element analysis for C₁₅H₁₁BrN₂OS:
   Calculated: C% 51.89 H% 3.19 N% 8.07 S% 9.23.
   Found: C% 51.71 H% 3.04 N% 7.98 S% 9.10.
- 6-Bromo-3-(2,6-difluorophenyl)-2-methylthio-4-oxo-3,4-dihydroquinazoline(TC.3.36):
   Obtained following the general methodology described above. Reagents: **TC.3.29** (100 mg, 0.27 mmol), K₂CO₃ (37.45 mg, 0.27 mmol), DMF (5.86 mL), methyl iodide (25.22 µL, 0.40 mmol). Reaction time: 4 hours. Purification: column chromatography using as eluent hexane/ethyl acetate (8:1). Product: white solid (102.40 mg, 99%).
   ¹H-RMN (300 MHz, CDCl₃), δ: 8.35 (d, 1H, *J*= 2.3 Hz, H-5); 7.82 (dd, 1H, *J*= 8.6 and 2.3 Hz, H-7); 7.58-7.43 (m, 1H, H-d); 7.51 (d, 1H, *J*= 8.6 Hz, H-8); 7.14-7.08 (m, 2H, H-c, H-c'); 2.57 (Me).
   ¹³C-RMN (75 MHz, CDCl₃), δ: 159.4 (C-4); 158.9 (dd, 2C, *J*= 253.7 and 3.4 Hz, C-b. C-b'); 157.8 (C-2); 146.6 (C-8a); 138.0 (C-7); 132.2 (t, 1C, *J*= 9.8 Hz, C-d); 129.8 (C-5); 128.2 (C-8); 120.6 (C-4a); 119.2 (C-6); 112.8 (t, 1C, *J*= 16.9 Hz, C-a); 112.4 (dd, 2C, *J*= 19.8 and 3.3 Hz, C-c, C-c'); 15.0 (Me).
   EM (ES): m/z= 383, 385 (M + H)⁺, 405, 407 (M + Na)⁺, 789 (2M + Na)⁺.
   Element analysis for C₁₅H₉BrF₂N₂OS:
   Calculated: C% 47.01 H% 2.37 N% 7.31 S% 8.37.
   Found: C% 47.31 H% 2.37 N% 7.24 S% 8.32.
- 6-Bromo-3-(2,3,4-trifluorophenyl)-2-methylthio-4-oxo-3,4-dihydroquinazoline(TC.3.32):
   Obtained following the general methodology described above. Reagents: **TC.3.30** (100 mg, 0.25 mmol), K₂CO₃ (35.71 mg, 0.25 mmol), DMF (5.60 mL), methyl iodide (24.10 µL, 0.38 mmol). Reaction time: 4 hours. Purification: column chromatography using as eluent hexane/ethyl acetate (10:1). Product: white solid (66.30 mg, 64%).
   ¹H-RMN (300 MHz, CDCl₃), δ: 8.32 (d, 1H, *J*= 2.3 Hz, H-5); 7.82 (dd, 1H, *J*= 8.6 and 2.3 Hz, H-7); 7.51 (d, 1H, *J*= 8.6 Hz, H-8); 7.21-7.06 (m, 2H, H-c, H-b); 2.56 (s, 3H, Me).
   ¹³C-RMN (75 MHz, CDCl₃), δ: 159.7 (C-4); 157.6 (C-2); 152.4 (ddd, 1C, *J*= 253.3, 9.9 and 2.9 Hz, C-d); 148.0 (ddd, 1C, *J*= 255.6, 10.9 and 4.3 Hz, C-b'); 146.5 (C-8a); 140.7 (ddd, 1C, *J*= 253.2, 15.6 and 13.8 Hz, C-c'); 138.1 (C-7); 129.7 (C-5); 128.2 (C-8); 124.9 (dd, 1C, *J*= 8.1 and 3.9 Hz, C-b); 120.5 (dd, 1C, *J*=11.1 and 3.9 Hz, C-a); 120.5 (C-4a); 119.3 (C-6); 112.4 (dd, 1C, *J*= 18.6 and 3.9 Hz, C-c); 15.7 (Me).
   EM (ES): m/z= 401, 403 (M + H)⁺, 423, 425 (M + Na)⁺. 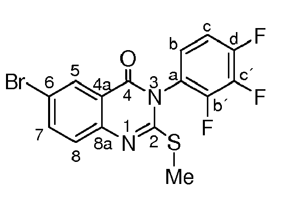
   Element analysis for C₁₅H₈BrF₃N₂OS:
   Calculated: C% 44.91 H% 2.01 N% 6.98 S% 7.99.
   Found: C% 44.71 H% 2.02 N% 6.69 S% 7.58.
- 6-Bromo-3-(2-bromophenyl)-2-methylthio-4-oxo-3,4 dihydroquinazoline(TC.3.35):
   Obtained following the general methodology described above. Reagents: **TC.3.28** (125 mg, 0.30 mmol), K₂CO₃ (41.93 mg, 0.30 mmol), DMF (6.58 mL), methyl iodide (28.30 µL, 0.45 mmol). Reaction time: 5 hours. Purification: column chromatography using as eluent hexane/ethyl acetate (6:1). Product: white solid (89.10 mg, 69%).
   ¹H-RMN (300 MHz, CDCl₃), δ: 8.39 (d, 1H, *J*= 2.3 Hz, H-5); 7.84 (dd, 1H, *J*= 8.6 and 2.3 Hz, H-7); 7.80 (dd, 1H, *J*= 7.8 and 1.3 Hz, H-c'); 7.55 (d, 1H, *J*= 8.6 Hz, H-8); 7.56-7.50 (m, 1H, H-c); 7.47-7.39 (m, 2H, H-b, H-d); 2.57 (s, 3H, Me).
   ¹³C-RMN (75 MHz, CDCl₃), δ: 159.7 (C-4); 158.0 (C-2); 146.7 (C-8a); 137.8 (C-7); 135.0 (C-a); 133.9 (C-c'); 131.6 (C-b); 130.9 (C-d); 129.8 (C-5); 128.7 (C-c); 128.1 (C-8); 123.7 (C-b'); 121.0 (C-4a); 119.0 (C-6); 15.3 (Me).
   EM (ES): m/z= 425, 427, 429 (M + H)⁺, 447, 449, 451 (M + Na)⁺, 875 (2M + Na)⁺. 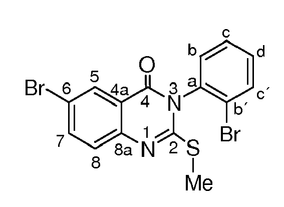
   Element analysis for C₁₅H₁₀Br₂N₂OS:
   Calculated: C% 42.28 H% 2.37 N% 6.57 S% 7.52.
   Found: C% 42.42 H% 2.49 N% 6.57 S% 7.23.
   HPLC: purity= 98%.
- 3-Phenyl-8-methyl-2-methylthio-4-oxo-3,4-dihydroquinazoline(TC.3.15b):
   Obtained following the general methodology described above. Reagents: **TC.2.31** (130 mg, 0.48 mmol), K₂CO₃ (67.04 mg, 0.48 mmol), DMF (10.54 mL), methyl iodide (45.30 µL, 0.72 mmol). Reaction time: 24 hours. Purification: column chromatography using as eluent hexane/ethyl acetate (8:1). Product: white solid (9,70 mg, 7%).
   ¹H-RMN (300 MHz, CDCl₃), δ: 8.09 (d, 1H, *J*= 7.9 Hz, H-5); 7.60 (dd, 1H, *J*= 7.2 and 0.6 Hz, H-7); 7.57-7.52 (m, 3H, H-c, H-c', H-d); 7.35-7.27 (m, 2H, H-b, H-b',H-6); 2.62 (s, 3H, Me); 2.54 (s, 3H, SMe).
   ¹³C-RMN (75 MHz, CDCl₃), δ: 162.1 (C-4); 156.6 (C-2); 146.2 (C-8a); 136.0 (C-a); 135.1 (C-7); 134.7 (C-8); 129.8 (C-d); 129.6 (2C, C-c, C-c'); 129.1 (2C, C-b, C-b'); 125.3 (C-6); 124.8 (C-5); 119.6 (C-4a); 17.2 (Me); 15.5 (SMe).
   EM (ES): m/z= 283 (M + H)⁺, 305 (M + Na)⁺, 587 (2M + Na)⁺.
   EM (IE): m/z= 282 (M⁺, 100). 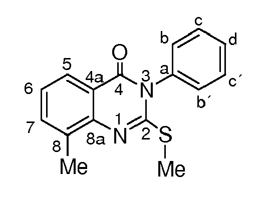
   Element analysis for C₁₆H₁₄N₂OS:
   Calculated: C% 68.06 H% 5.00 N% 9.92 S% 11.36.
   Found: C% 67.89 H% 4.78 N% 9.94 S% 11.18.
- 3-(2,6-Difluorophenyl)-8-methyl-2-methylthio-4-oxo-3,4-dihydroquinazoline(TC.3.23):
   Obtained following the general methodology described above. Reagents: **TC.3.16** (100 mg, 0.32 mmol), K₂CO₃ (45.46 mg, 0.32 mmol), DMF (7.13 mL), methyl iodide (30.64 µL, 0.49 mmol). Reaction time: 4 hours. Purification: column chromatography using as eluent hexane/ethyl acetate (5:1). Product: white solid (59.50 mg, 57%).
   ¹H-RMN (300 MHz, CDCl₃), δ: 8.10 (dd, 1H, *J*= 7.9 and 0.7 Hz, H-5); 7.61 (dd, 1H, *J*= 7.3 and 0.7 Hz, H-7); 7.56-7.46 (m, 1H, H-d); 7.31 (dd, 1H, *J*= 7.9 and 7.3 Hz, H-6); 7.14-7.08 (m, 2H, H-c, H-c'); 2.62 (s, 3H, Me); 2.60 (s, 3H, SMe).
   ¹³C-RMN (75 MHz, CDCl₃), δ: 161.0 (C-4); 159.0 (dd, 2C, *J*= 253.5 and 3.6 Hz, C-b, C-b'); 155.7 (C-2); 146.1 (C-8a); 135.5 (C-7); 134.9 (C-8); 131.9 (t, 1C, *J*= 9.8 Hz, C-d); 125.5 (C-6); 124.9 (C-5); 119.1 (C-4a); 113.1 (t, 1C, *J*= 16.9 Hz, C-a); 112.3 (dd, 2C, *J*= 19.9 and 3.3 Hz, C-c, C-c'); 17.1 (Me); 15.0 (SMe).
   EM (IE): m/z= 318 (M⁺, 100). 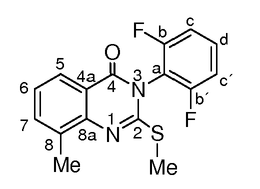
   Element analysis for C₁₆H₁₂F₂N₂OS:
   Calculated: C% 60.37 H% 3.80 N% 8.80 S% 10.07.
   Found: C% 60.18 H% 3.65 N% 8.62 S% 9.78.
- 3-(2,3,4-Trifluorophenyl)-8-methyl-2-methylthio-4-oxo-3,4-dihydroquinazoline(TC.3.24):
   Obtained following the general methodology described above. Reagents: **TC.3.17** (75 mg, 0.23 mmol), K₂CO₃ (32.19 mg, 0.23 mmol), DMF (5.06 mL), methyl iodide (21.67 µL, 0.34 mmol). Reaction time: 5 hours. Purification: column chromatography using as eluent hexane/ethyl acetate (6:1). Product: white solid (43.50 mg, 56%).
   ¹H-RMN (300 MHz, CDCl₃), δ: 8.08 (dd, 1H, *J*= 7.9 and 0.7 Hz, H-5); 7.61 (d, 1H, *J*= 7.3 Hz, H-7); 7.31 (dd, 1H, *J*= 7.9 and 7.3 Hz, H-6); 7.19-7.07 (m, 2H, H-c, H-b); 2.61 (s, 3H, Me); 2.59 (s, 3H, SMe).
   ¹³C-RMN (75 MHz, CDCl₃), δ: 161.3 (C-4); 155.5 (C-2); 152.3 (ddd, 1C, *J*= 252.9, 9.9 and 2.2 Hz, C-d); 148.2 (ddd, 1C, *J*= 254.2, 11.5 and 4.5 Hz, C-b'); 146.0 (C-8a); 140.7 (ddd, 1C, *J*= 252.9, 15.6 and 14.1 Hz, C-c'); 135.7 (C-7); 134.9 (C-8); 125.7 (C-6); 125.0 (dd, C, *J*= 8.1 and 3.9 Hz, C-b); 124.9 (C-5); 121.0 (dd, 1C, *J*= 11.2 and 3.9 Hz, C-a); 119.1 (C-4a); 112.3 (dd, 1C, *J*= 18.5 and 3.8 Hz, C-c); 17.1 (Me); 15.2 (SMe).
   EM (IE): m/z= 336 (M⁺, 100). 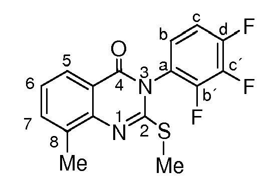
   Element analysis for C₁₆H₁₁F₃N₂OS:
   Calculated: C% 57.14 H% 3.30 N% 8.33 S% 9.53.
   Found: C% 57.35 H% 3.46 N% 8.23 S% 9.21.
- 3-(2-Bromophenyl)-8-methyl-2-methylthio-4-oxo-3,4-dihydroquinazoline(TC.3.22):
   Obtained following the general methodology described above. Reagents: **TC.3.14** (250 mg, 0.72 mmol), K₂CO₃ (99.57 mg, 0.72 mmol), DMF (15.65 mL), methyl iodide (67.30 µL, 1.08 mmol). Reaction time: 6 hours. Purification: column chromatography using as eluent hexane/ethyl acetate (5:1). Product: white solid (220.40 mg, 85%).
   ¹H-RMN (300 MHz, CDCl₃), δ: 8.12 (d, 1H, *J*= 7.9 Hz, H-5); 7,77 (dd, 1H, *J*= 7.5 and 1.3 Hz, H-c'); 7.61 (d, 1H, *J*= 7.2 Hz, H-7); 7.50 (td, 1H, *J*= 7.5 and 1.3 Hz, H-c); 7.45-7.37 (m, 2H, H-b, H-d); 7.30 (dd, 1 H, *J*= 7.9 and 7.2 Hz, H-6); 2.64 (s, 1H, Me); 2.58 (s, 1H, SMe).
   ¹³C-RMN (75 MHz, CDCl₃), δ: 161.3 (C-4); 155.8 (C-2); 146.2 (C-8a); 135.4 (C-a); 135.3 (C-7); 134.7 (C-8); 133.8 (C-c'); 131.4 (C-b); 131.0 (C-d); 128.6 (C-c); 125.4 (C-6); 124.9 (C-5); 123.9 (C-b'); 119.4 (C-4a); 17.2 (Me); 15.3 (SMe).
   EM (IE): m/z= 266 (M⁺- Br - Me, 47), 281 (M⁺- Br, 100), 360, 362 (M⁺, 8, 9). 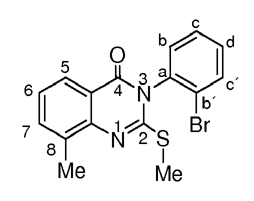
   Element analysis for C₁₆H₁₃BrN₂OS:
   Calculated: C% 53.20 H% 3.63 N% 7.75 S% 8.88.
   Found: C% 52.90 H% 3.48 N% 7.75 S% 8.59.
   HPLC: purity= 99%.

### Example 3.- General procedure for obtaining the derivatives of 2,4-dithioxo-1,2,3,4-tetrahydroquinazoline (Compounds Ic, Figure 3)

To a solution of the derivative of 4-oxo-2-thioxo-1,2,3,4-tetrahydroquinazoline in corresponding toluene the Lawesson's reagent is added (1.5 equivalents) and is agitated to reflux. Next, the reaction mixture is allowed to cool to room temperature and the solvent is eliminated through evaporation at reduced pressure. The solid obtained is purified as indicated in each case.
- 3-Phenyl-2,4-dithioxo-1,2,3,4-tetrahydroquinazoline (TC.3.6):
   Obtained following the general methodology described above. Reagents: 3-phenyl-4-oxo-2-thioxo-1,2,3,4-tetrahydroquinazoline **TC.3.5** (75 mg, 0.29 mmol), Lawesson's reagent (178.93 mg, 0.44 mmol), toluene (6.41 mL). Reaction time: 24 hours. Purification: column chromatography using as eluent hexane/ethyl acetate (9:1). Product: orange solid (50.30 mg, 63%).
   ¹H-RMN (300 MHz, DMSO-*d*₆), δ: 13.48 (sa, 1H, NH); 8.32 (dd, 1H, *J*= 8.2 and 1.0 Hz, H-5); 7.78 (ddd, 1H, *J*= 8.3, 7.0 and 1.0 Hz, H-7); 7.50-7.42 (m, 3H, H-c, H-c', H-6); 7.39-7.32 (m, 2H, H-d, H-8); 7.21 (dd, 1H, *J*= 8.3 and 1.1 Hz, H-b, H-b').
   ¹³C-RMN (75 MHz, DMSO-*d*₆), δ: 189.8 (C-4); 172.8 (C-2); 144.2 (C-a); 135.8 (C-7); 135.2 (C-8a); 131.7 (C-5); 129.2 (2C, C-c, C-c'); 128.5 (2C, C-b, C-b'); 127.9 (C-d); 125.2 (C-8); 123.7 (C-4a); 115.9 (C-6). 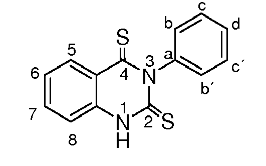
   EM (ES): m/z= 271 (M + H)⁺, 293 (M + Na)⁺.
   EM (IE): m/z= 270 (M⁺, 60).
   HPLC: purity= 98%.
- 3-(2,6-Difluorophenyl)-2,4-dithioxo-1,2,3,4-tetrahydroquinazoline (TC.2.49):
   Obtained following the general methodology described above. Reagents: 3-(2,6-difluorophenyl)-4-oxo-2-thioxo-1,2,3,4-tetrahydroquinazoline **TC.2.40** (100 mg, 0.34 mmol), Lawesson's reagent (208.96 mg, 0.51 mmol), toluene (7.49 mL). Reaction time: 4 days. Purification: column chromatography using as eluent hexane/ethyl acetate (8:1). Product: orange solid (42,80 mg, 41 %).
   ¹H-RMN (300 MHz, DMSO-*d*₆), δ: 13.83 (sa, 1H, NH); 8.33 (dd, 1H, *J*= 8.2 and 1.3 Hz, H-5); 7.87 (ddd, 1H, *J*= 8.4, 7.4 and 1.3 Hz, H-7); 7.64-7.54 (m, 1H, H-d); 7.46 (dd, 1H, *J*= 8.4 and 0.9 Hz, H-8); 7.41 (ddd, 1H, *J*= 8.2, 7.4 and 0.9 Hz, H-6); 7.33-7.26 (m, 2H, H-c, H-c').
   ¹³C-RMN (75 MHz, DMSO-*d*₆), δ: 188.2 (C-4); 171.4 (C-2); 156.1 (dd, 2C, *J*= 247.8 and 4.8 Hz, C-b, C-b'); 136.7 (C-7); 135.0 (C-8a); 131.4 (C-5); 131.1 (t, 1C, *J*= 9.8 Hz, C-d); 125.8 (C-6); 122.3 (C-4a); 119.7 (t, 1C, *J*= 16.5 Hz, C-a); 116.2 (C-8); 112.2 (dd, 2C, *J*= 19.4 and 3.0 Hz, C-c, C-c').
   EM (IE): m/z= 306 (M⁺, 8). 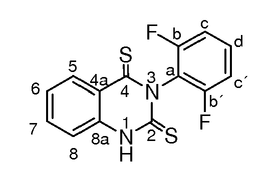
   Element analysis for C₁₄H₈F₂N₂S₂:
   Calculated: C% 54.89 H% 2.63 N% 9.14 S% 20.93.
   Found: C% 54.65 H% 2.91 N% 9.32 S% 20.78.
- 3-(2,3,4-Trifluorophenyl)-2,4-dithioxo-1,2,3,4-tetrahydroquinazoline (TC.2.45):
   Obtained following the general methodology described above. Reagents: 3-(2,3,4-trifluorophenyl)-4-oxo-2-thioxo-1,2,3,4-tetrahydroquinazoline **TC.2.39** (100 mg, 0.32 mmol), Lawesson's reagent (196.75 mg, 0.48 mmol), toluene (7.05 mL). Reaction time: 24 hours. Purification: column chromatography using as eluent hexane/ethyl acetate (4:1). Product: orange solid (44.00 mg, 42%).
   ¹H-RMN (300 MHz, DMSO-*d*₆), δ: 13.74 (sa, 1H, NH); 8.32 (d, 1H, *J*= 8.2 Hz, H-5); 7.84 (ddd, 1H, *J*= 8.2, 7.2 and 1.2 Hz, H-7); 7.56-7.37 (m, 4H, H-6, H-8, C-b, C-c).
   ¹³C-RMN (75 MHz, DMSO-*d*₆), δ: 189.4 (C-4); 172.1 (C-2); 150.3 (dd, 1C, *J*= 247.2 and 8.1 Hz, C-d); 146.0 (dd, 1C, *J*= 245.1 and 10.0 Hz, C-b'); 139.7 (ddd, 1C, *J*= 246.3, 15.6 and 14.1 Hz, C-c'); 136.5 (C-7); 135.3 (C-8a); 131.6 (C-5); 128.8 (dd, 1 C, *J*= 10.2 and 3.9 Hz, C-a); 125.8 (C-8); 125.5 (dd, 1C, *J*= 8.7 and 3.4 Hz, C-b); 123.2 (C-4a); 116.2 (C-6); 113.1 (dd, 1C, *J*= 18.2 and 3.4 Hz, C-c).
   EM (IE): m/z= 324 (M⁺, 13). 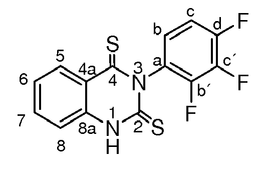
   Element analysis for C₁₄H₇F₃N₂S₂:
   Calculated: C% 51.84 H% 2.18 N% 8.64 S% 19.77.
   Found: C% 51.67 H% 2.23 N% 8.83 S% 19.98.

### Example 4.- General procedure for obtaining the derivatives of 2-methylthio-4-thioxo-3,4-dihydroquinazoline (Compounds Id, Figure 4)

To a solution of the derivative of 2-methylthio-4-oxo-3,4-dihydroquinazolinecorrespondiente in toluene (1 equivalent) Lawesson's reagent is added (1.5 equivalents) and agitated to reflux. Then, the reaction mixture is allowed to cool to room temperature and the solvent is eliminated through evaporation at reduced pressure. The solid obtained is purified as indicated in each case.
- 3-Phenyl-2-methylthio-4-thioxo-3,4-dihydroquinazoline(TC.3.9):
   Obtained following the general methodology described above. Reagents: 3-phenyl-2-methylthio-4-thioxo-3,4-dihydroquinazoline TC.3.7 (75 mg, 0.27 mmol), Lawesson's reagent (169.58 mg, 0.41 mmol), toluene (6.08 mL). Reaction time: 24 hours. Purification: column chromatography using as eluent hexane/ethyl acetate (6:1). Product: yellow solid (73.90 mg, 93%).
   ¹H-RMN (300 MHz, CDCl₃), δ: 8.68 (ddd, 1H, *J*= 8.2, 1.3 and 0.3 Hz, H-5); 7.70 (ddd, 1 H, *J*= 8.2, 6.9 and 1.3 Hz, H-7); 7.59 (dd, 1H, *J*= 8.2 and 0.9 Hz, H-8); 7.56-7.51 (m, 3H, H-d, H-b, H-b'); 7.37 (ddd, 1H, *J*= 8.2, 6.9 and 0.9 Hz, H-6); 7.26-7.22 (m, 2H, H-c, H-c'); 2.49 (s, 3H, Me).
   ¹³C-RMN (75 MHz, CDCl₃), δ: 189.9 (C-4); 157.4 (C-2); 142.8 (C-8a); 140.4 (C-a); 134.8 (C-7); 131.1 (C-5); 129.9 (C-d); 129.8 (2C, C-b, C-b'); 128.8 (2C, C-c, C-c'); 127.5 (C-4a); 126.9 (C-6); 126.8 (C-8); 16.0 (Me).
   EM (ES): m/z= 285 (M + H)⁺, 307 (M + Na)⁺, 591 (2M + Na)⁺. 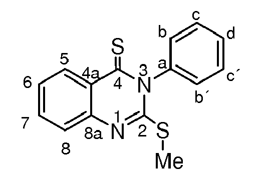
   Element analysis for C₁₅H₁₂N₂S₂:
   Calculated: C% 63.35 H% 4.25 N% 9.85 S% 22.55.
   Found: C% 63.58 H% 4.76 N% 9.83 S% 22.30.
   HPLC: purity= 98%.
- 3-(2,6-difluorophenyl)-2-methylthio-4-thioxo-3,4-dihydroquinazoline(TC.3.1):
   Obtained following the general methodology described above. Reagents: 3-(2,6-difluorophenyl)-2-methylthio-4-thioxo-3,4-dihydroquinazoline **TC.2.43** (75 mg, 0.24 mmol), Lawesson's reagent (149.50 mg, 0.37 mmol), toluene (5.36 mL). Reaction time: 8 hours. Purification: column chromatography using as eluent hexane/ethyl acetate (7:1). Product: yellow solid (48.00 mg, 61 %).
   ¹H-RMN (300 MHz, CDCl₃), δ: 8.71 (ddd, 1H, *J*= 8.2, 1.3 and 0.5 Hz, H-5); 7.77 (ddd, 1H, *J*= 7.9, 7.1 and 1.3 Hz, H-7); 7.64 (ddd, 1H, *J*= 7.9, 1.1 and 0.5 Hz, H-8); 7.58-7.52 (m, 1H, H-d); 7.43 (ddd, 1H, *J*= 8.2, 7.1 and 1.1 Hz, H-6); 7.16-7.10 (m, 2H, H-c, H-c'); 2.59 (s, 3H, Me).
   ¹³C-RMN (75 MHz, CDCl₃), δ: 189.2 (C-4); 158.2 (dd, 2C, *J*= 253.7 and 4.0 Hz, C-b, C-b'); 156.6 (C-2); 142.8 (C-8a); 135.2 (C-7); 132.2 (t, 1C, *J*= 9.7 Hz, C-d); 131.1 (C-5); 127.1 (C-6); 127.1 (C-4a); 126.9 (C-8); 117.2 (t, 1C, *J*= 16.5 Hz, C-a); 112.5 (dd, 2C, *J*= 19.3 and 3.5 Hz, C-c, C-c'); 15.5 (Me).
   EM (ES): m/z= 321 (M + H)⁺. 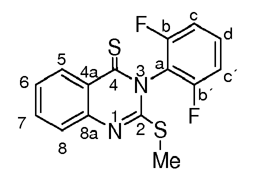
   Element analysis for C₁₅H₁₀F₂N₂S₂:
   Calculated: C% 56.23 H% 3.15 N% 8.74 S% 20.02.
   Found: C% 55.98 H% 3.56 N% 8.45 S% 19.92.
- 3-(2,3,4-trifluorophenyl)-2-methylthio-4-thioxo-3,4-dihydroquinazoline(TC.2.51):
   Obtained following the general methodology described above. Reagents: 3-(2,3,4-trifluorophenyl)-2-methylthio-4-thioxo-3,4-dihydroquinazoline **TC.2.41** (75 mg, 0.23 mmol), Lawesson's reagent (141.14 mg, 0.34 mmol), toluene (5.06 mL). Reaction time: 7 hours. Purification: column chromatography using as eluent hexane/ethyl acetate (6:1). Product: yellow solid (46.80 mg, 60%).
   ¹H-RMN (300 MHz, CDCl₃), δ: 8.68 (ddd, 1H, *J*= 8.2, 1.3 and 0.4 Hz, H-5); 7.77 (ddd, 1H, *J*= 8.1, 7.1 and 1.3 Hz, H-7); 7.64 (dd, 1H, *J*= 8.1 and 0.9 Hz, H-8); 7.44 (ddd, 1H, *J*= 8.2, 7.1 and 0.9 Hz, H-6); 7.23-7.13 (m, 1H, H-c); 7.11-7.04 (m, 1H, H-b); 2.49 (s, 3H, Me).
   ¹³C-RMN (75 MHz, CDCl₃), δ: 189.6 (C-4); 156.5 (C-2); 152.4 (ddd, 1C, *J*= 253.2, 9.8 and 3.0 Hz, C-d); 147.6 (ddd, 1C, *J*= 255.5, 10.9 and 4.1 Hz, C-b'); 142.6 (C-8a); 141.0 (ddd, 1C, *J*= 253.0, 15.9 and 13.5 Hz, C-c'); 135.3 (C-7); 131.1 (C-5); 127.2 (C-6); 127.2 (C-4a); 126.9 (C-8); 124.9 (dd, 1C, *J*= 10.5 and 4.1 Hz, C-a); 124.6 (dd, 1C, *J*= 8.2 and 4.0 Hz, C-b); 112.7 (dd, 1C, *J*= 18.6 and 3.8 Hz, C-c); 15.8 (Me). 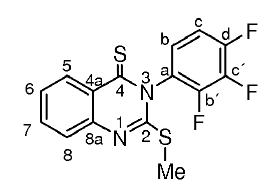
   EM (ES): m/z= 319 (M - F)⁺, 339 (M + H)⁺, 677 (2M + H)⁺.
   Element analysis for C₁₅H₉F₃N₂S₂:
   Calculated: C% 53.24 H% 2.68 N% 8.28 S% 18.95.
   Found: C% 53.28 H% 2.73 N% 8.87 S% 18.99.
- 3-(2-bromophenyl)-2-methylthio-4-thioxo-3,4-dihydroquinazoline(TC.3.13):
   Obtained following the general methodology described above. Reagents: 3-(2-bromophenyl)-2-methylthio-4-thioxo-3,4-dihydroquinazoline **TC.3.11** (70 mg, 0.20 mmol), Lawesson's reagent (122.24 mg, 0.30 mmol), toluene (4.38 mL). Reaction time: 6 days. Purification: column chromatography using as eluent hexane/ethyl acetate (5:1). Product: yellow solid (32.30 mg, 45%).
   ¹H-RMN (300 MHz, CDCl₃), δ: 8.73 (dd, 1H, *J*= 8.2 and 0.9 Hz, H-5); 7.79-7.73 (m, 2H, H-c', H-7); 7.65 (d, 1H, *J*= 8.1 Hz, H-8); 7.53 (td, 1H, *J*= 7.5 and 1.3 Hz, H-c); 7.45-7.35 (m, 3H, H-b, H-6, H-d); 2.57 (s, 3H, Me).
   ¹³C-RMN (75 MHz, CDCl₃), δ: 188.8 (C-4); 156.8 (C-2); 142.8 (C-8a); 139.2 (C-a); 135.0 (C-7); 134.0 (C-c'); 131.3 (C-6); 131.1 (C-5); 130.8 (C-d); 128.9 (C-c); 127.4 (C-4a); 127.0 (C-b); 126.9 (C-8); 123.2 (C-b'); 15.9 (Me).
   EM (ES): m/z= 283 (M - Br)⁺. 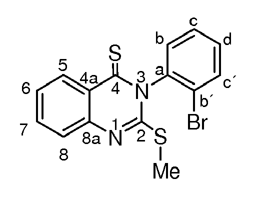
   Element analysis for C₁₅H₁₁BrN₂S₂:
   Calculated: C% 49.59 H% 3.05 N% 7.71 S% 17.65.
   Found: C% 49.87 H% 3.15 N% 7.37 S% 17.25.
   HPLC: purity= 95%.

### Example 5.- Biological studies

### PDE inhibitor test

The catalytic domain of PDE7A1 (aá. 130-482) and the complete enzyme of PDE4D2 (aa. 1-507) were subcloned in pET vectors, overexpressed in E.coli strain BL21, and purified to homogeneity as described in the bibliography (Wang, H., Liu, Y., Chen, Y., Robinson, H., and Ke, H. Multiple elements jointly determine inhibitor selectivity of cyclic nucleotide phosphodiesterases 4 and 7. J. Biol. Chem. 2005, 280, 30949-30955). The enzymatic activities of PDE7A1 and PDE4D2 were tested through incubation of the enzymes with 100 µl of a reaction mixture that contained 50 mM Tris•HCl (pH 8.5), 50 mM MgCl₂, 5 mM DTT, and ³H-cAMP (20000 cpm per test, Sigma-Aldrich) at room temperature during 10 minutes. The reactions were concluded by adding 200 µl 0.2 M ZnSO₄ when 20-50% of the cAMP was hydrolysed. The product of the reaction, ³H-AMP, was precipitated by adding 200 µl 0.25 M Ba(OH)₂ (Sigma-Aldrich), while the unreacted ³H-AMPc remained in the supernatant. Radioactivity of the supernatant was measured with a mixture of scintillation liquid (ScintiSafe PlusTM 50%, Fisher Scientific) using the counter LKB RackBeta 1214.

For inhibitor measurements, six concentrations of inhibitors were used in the presence of a substrate concentration equivalent to 1/10 of the K_{M} and the enzyme concentration that hydrolyses 50% of the substrate. The CI50 is defined as the compound concentration that inhibits the enzymatic activity by 50%. All experiments were carried out in duplicate.

**Table 1. Inhibitor data of PDE7A1 and PDE4D2**

| Comp. | PDE7A1 | PDE4D2 |
|---|---|---|
| **TC2.30A** | >10 µM | N.D. |
| **TC3.29** | >10 µM | N.D. |
| **TC3.30** | >10 µM | N.D. |
| **TC3.28** | ~10 µM | N.D. |
| **TC3.16** | >1 µM | N.D. |
| **TC3.17** | >10 µM | N.D. |
| | | |
| **TC3.14** | 1.70±0.10µM | 34.00±5.00µM |
| **TC3.47A** | >1 µM | N.D. |
| **TC3.36** | 0.24±0.03µM | 4.50±0.10µM |
| **TC3.32** | 2.10±0.10µM? | N.D. |
| **TC3.35** | 1.86±0.18µM | N.D. |
| **TC3.15b** | ~1 µM | N.D. |
| **TC3.23** | 0.13±0.02µM | 1.40±0.20µM |
| **TC3.24** | >1 µM | N.D. |
| **TC3.22** | 0.27±0.02µM | 1.10±0.20µM |
| **TC2.43** | 0.51±0.02µM | 3.50±0.30µM |
| **TC2.41** | ~10 µM | N.D. |
| **TC3.6** | 1.04±0.08µM | 5.70±0.03µM |
| **TC2.49** | ~1 µM | N.D. |
| **TC2.45** | >1 µM | N.D. |
| **TC3.9** | 0.84±0.01µM | 8.00±1.20µM |
| **TC3.1** | 0.1-1µM | N.D. |
| **TC2.51** | >1 µM | N.D. |
| **TC3.13** | 0.1-1µM | N.D. |

### Cell toxicity measurements

At the same time, measurements of the cell toxicity of the compounds of formula (I) of the invention were taken.

Cell toxicity tests were carried out on a clone of Th2 lymphocytes (D10.G4.1) through flow cytometry using Propidium lodide in order to determine cell death. This technique is based on quantifying the cells in which Propidium lodide can penetrate because their membrane is damaged, while whole or intact cells do not allow this fluorochrome which intersperses in the nucleic acids to pass.

The cell concentration used was 1x10⁶ cells/ml, in a final volume of 200µl, in the presence of different concentrations of the inhibitors during 48h. After this time, the cells were washed and incubated with 5µg/ml of Propidium lodide during 1 min. The samples treated in this way were analysed by cytometry using BD FACSCanto™ equipment. These experiments were carried out in parallel with two commercial inhibitors: Rolipram (inhibitor of PDE4) and BRL 50481 (inhibitor of PDE7).

From the analysis of the compounds of new synthesis it can be deduced that those with the most moderate toxicity are: TC3.6, TC3.14 and TC3.15.

**Table 2 For each inhibitor the maximum concentration at which more than 80% cellular viability was maintained is shown in comparison to the control of cells without inhibitor.**

| **Compound** | **Concentration (µM)** |
|---|---|
| **BRL50481** | >1000 |
| **Rolipram** | >1000 |
| **TC3.6** | 1000 |
| **TC3.15** | 600 |
| **TC3.14** | 600 |
| **TC3.9** | 100 |
| **TC3.1** | 10 |
| **TC3.32** | 100 |
| **TC2.49** | 50 |
| **TC3.23** | 10 |
| **TC3.13** | 10 |
| **TC3.36** | 10 |
| **TC3.35** | 10 |
| **TC3.22** | 5 |
| **TC2.43** | 5 |

### Study of intracellular cAMP levels

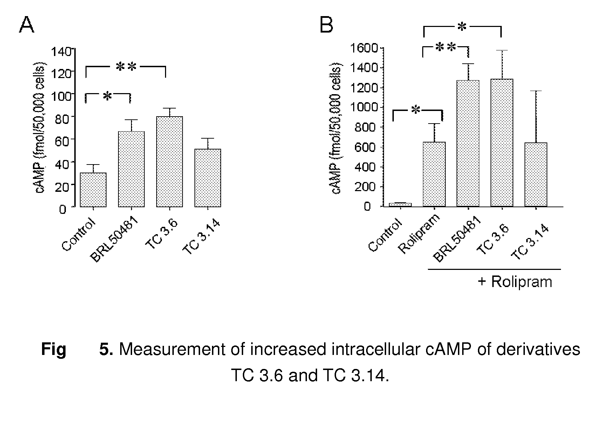

PDE inhibition has the direct effect of increasing cAMP, since their degradation is prevented. In order to verify this effect on the cell system of T-Iymphocytes two compounds of formula (I) of the invention were chosen, specifically: TC 3.6 and TC 3.14 and cAMP concentration was measured using the cAMP *Biotrak Enzymeimmunoassay System of Amersham Biosciences* kit. As can be observed in Figure 5, the amount of intracellular cAMP increased in a statistically significant manner with the inhibitors of PDE7 in relation to the control, although a greater increase is observed with the combination of Rolipram and the PDE7 inhibitors, with a synergy effect occurring that considerably increased the levels of detected intracellular cAMP through inhibition of PDE4.

Determination of the levels of intracellular cAMP in T-lymphocytes treated with these inhibitors has demonstrated that the inhibition of PDE7 does not produce high levels of cAMP, but is capable of synergising the effect produced by the inhibition of PDE4.

## Claims

1. Compound with a dual activity inhibitory of enzymes PDE7 and/or PDE4 **characterised in that** it presents the formula (I): 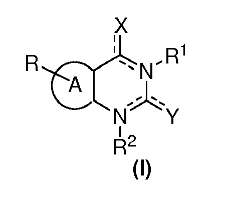 wherein:
A , is a carbocycle or optionally substituted fused heterocycle of 5, 6 or 7 members, saturated or unsaturated,
--- may be a double bond;
X and Y, are selected independently from among the group consisting of hydrogen, alkyl, =O, =S, -N(alkyl), -N(aryl), aryl, O-alkyl, O-aryl, S-alkyl or -S-aryl; and
R, R¹ and R², are selected independently from among the group consisting of hydrogen, halogen, alkyl, haloalkyl, aril, cycloalkyl, (Z)ₙ-aryl, heteroaryl, -OR³; -C(O)OR³, -(Z)ₙ-C(O)OR³ or -S(O)ₜ-,
or a pharmaceutically acceptable salt, derivative, solvate or stereoisomer thereof.
Exception: when A is benzene without substituting, X=O,Y=S
when A is benzene without substituting, X=O,Y=O,
when A is benzene without substituting, X=O,Y=S-Me
when A is thiophene without substituting, X=O,Y=S, and
when A is benzothiophene without substituting, X=O,Y=S

2. Compound according to claim 1 **characterised in that** it presents the formula 4-oxo-2-thioxo-1,2,3,4-tetrahydroquinazoline (Compound la).

3. Compound according to claim 2 **characterised in that** it belongs to the following group:
- 6-Bromo-3-phenyl-4-oxo-2-thioxo-1,2,3,4-tetrahydroquinazoline,
- 6-Bromo-3-(2,6-difluorophenyl)-4-oxo-2-thioxo-1,2,3,4-tetrahydroquinazoline,
- 6-Bromo-3-(2,3,4-trifluorophenyl)-4-oxo-2-thioxo-1,2,3,4-tetrahydroquinazoline,
- 6-Bromo-3-(2-bromophenyl)-4-oxo-2-thioxo-1,2,3,4-tetrahydroquinazoline,
- 3-(2,6-Difluorophenyl)-8-methyl-4-oxo-2-thioxo-1,2,3,4-tetrahydroquinazoline,
- 3-(2,3,4-Trifluorophenyl)-8-methyl-4-oxo-2-thioxo-1,2,3,4-tetrahydroquinazoline, y
- 3-(2-Bromophenyl)-8-methyl-4-oxo-2-thioxo-1,2,3,4-tetrahydroquinazoline.

4. Compound according to claim 1 **characterised in that** it presents the formula 2-methylthio-4-oxo-3,4-dihydroquinazoline (Compound Ib).

5. Compound according to claim 4 **characterised in that** it belongs to the following group:
- 6-Bromo-3-phenyl-2-methylthio-4-oxo-3,4-dihydroquinazoline,
- 6-Bromo-3-(2,6-difluorophenyl)-2-methylthio-4-oxo-3,4-dihydroquinazoline,
- 6-Bromo-3-(2,3,4-trifluorophenyl)-2-methylthio-4-oxo-3,4-dihydroquinazoline,
- 6-Bromo-3-(2-bromophenyl)-2-methylthio-4-oxo-3,4-dihydroquinazoline,
- 3-Phenyl-8-methyl-2-methylthio-4-oxo-3,4-dihydroquinazoline,
- 3-(2,6-Difluorophenyl)-8-methyl-2-methylthio-4-oxo-3,4-dihydroquinazoline, and
- 3-(2,3,4-Trifluorophenyl)-8-methyl-2-methylthio-4-oxo-3,4-dihydroquinazoline, and
- 3-(2-Bromophenyl)-8-methyl-2-methylthio-4-oxo-3,4-dihydroquinazoline.

6. Compound according to claim 1 **characterised in that** it presents the formula 2,4-dithioxo-1,2,3,4-tetrahydroquinazoline (Compound Ic).

7. Compound according to claim 6 **characterised in that** it belongs to the following group:
- 3-Phenyl-2,4-dithioxo-1,2,3,4-tetrahydroquinazoline,
- 3-(2,6-Difluorophenyl)-2,4-dithioxo-1,2,3,4-tetrahydroquinazoline, and
- 3-(2,3,4-Trifluorophenyl)-2,4-dithioxo-1,2,3,4-tetrahydroquinazoline.

8. Compound according to claim 1 **characterised in that** it presents the formula 2-methylthio-4-thioxo-3,4-dihydroquinazoline (Compound Id).

9. Compound according to claim 8 **characterised in that** it belongs to the following group:
- 3-Phenyl-2-methylthio-4-thioxo-3,4-dihydroquinazoline,
- 3-(2,6-difluorophenyl)-2-methylthio-4-thioxo-3,4-dihydroquinazoline,
- 3-(2,3,4-trifluorophenyl)-2 methylthio-4-thioxo-3,4-dihydroquinazoline, and
- 3-(2-bromophenyl)-2-methylthio-4-thioxo-3,4-dihydroquinazoline.

10. Pharmaceutical composition **characterised in that** it comprises a compound, in a therapeutically effective quantity, of formula (I), or mixtures thereof, a pharmaceutically acceptable salt, derivative, prodrug, solvate or stereoisomer thereof together with a pharmaceutically acceptable carrier, adjuvant, or vehicle, for administration to a patient.

11. Pharmaceutical composition according to claim 10 **characterised in that** the compound of formula (I) belongs to one, or to several, of the following families of compounds:
- 4-oxo-2-thioxo-1,2,3,4-tetrahydroquinazoline (Compound la),
- 2-methylthio-4-oxo-3,4-dihydroquinazoline (Compound Ib),
- 2,4-dithioxo-1,2,3,4-tetrahydroquinazoline (Compound Ic), and
- 2-methylthio-4-thioxo-3,4-dihydroquinazoline (Compound Id),
or any of their R, S enantiomeric forms and/or racemic mixtures.

12. Pharmaceutical composition according to claim 11 **characterised in that** the compound or mixture of compounds of formula (I) belong to the following group:
- 6-Bromo-3-phenyl-4-oxo-2-thioxo-1,2,3,4-tetrahydroquinazoline,
- 6-Bromo-3-(2,6-difluorophenyl)-4-oxo-2-thioxo-1,2,3,4-tetrahydroquinazoline,
- 6-Bromo-3-(2,3,4-trifluorophenyl)-4-oxo-2-thioxo-1,2,3,4-tetrahydroquinazoline,
- 6-Bromo-3-(2-bromophenyl)-4-oxo-2-thioxo-1,2,3,4-tetrahydroquinazoline,
- 3-(2,6-Difluorophenyl)-8-methyl-4-oxo-2-thioxo-1,2,3,4-tetrahydroquinazoline,
- 3-(2,3,4-Trifluorophenyl)-8-methyl-4-oxo-2-thioxo-1,2,3,4-tetrahydroquinazoline,
- 3-(2-Bromophenyl)-8-methyl-4-oxo-2-thioxo-1,2,3,4-tetrahydroquinazoline,
- 6-Bromo-3-phenyl-2-methylthio-4-oxo-3,4-dihydroquinazoline,
- 6-Bromo-3-(2,6-difluorophenyl)-2-methylthio-4-oxo-3,4-dihydroquinazoline,
- 6-Bromo-3-(2,3,4-trifluorophenyl)-2-methylthio-4-oxo-3,4-dihydroquinazoline,
- 6-Bromo-3-(2-bromophenyl)-2-methylthio-4-oxo-3,4-dihydroquinazoline,
- 3-Phenyl-8-methyl-2-methylthio-4-oxo-3,4-dihydroquinazoline,
- 3-(2,6-Difluorophenyl)-8-methyl-2-methylthio-4-oxo-3,4-dihydroquinazoline,
- 3-(2,3,4-trifluorophenyl)-8-methyl-2-methylthio-4-oxo-3,4-dihydroquinazoline,
- 3-(2-bromophenyl)-8-methyl-2-methylthio-4-oxo-3,4-dihydroquinazoline,
- 3-Phenyl-2,4-dithioxo-1,2,3,4-tetrahydroquinazoline,
- 3-(2,6-Difluorophenyl)-2,4-dithioxo-1,2,3,4-tetrahydroquinazoline,
- 3-(2,3,4-Trifluorophenyl)-2,4-dithioxo-1,2,3,4-tetrahydroquinazoline.
- 3-Phenyl-2-methylthio-4-thioxo-3,4-dihydroquinazoline,
- 3-(2,6-difluorophenyl)-2-methylthio-4-thioxo-3,4-dihydroquinazoline,
- 3-(2,3,4-trifluorophenyl)-2 methylthio-4-thioxo-3,4-dihydroquinazoline, and
- 3-(2-bromophenyl)-2-methylthio-4-thioxo-3,4-dihydroquinazoline.

13. Use of a compound according to claims 1 to 9, or mixtures thereof, for the preparation of a medicine directed at treating inflammatory or autoimmune pathologies or diseases.

14. Use of a compound according to claim 13 **characterised in that** the inflammatory or autoimmune disease belongs to the following group: inflammatory intestinal disease, inflammatory joint pathologies, atopic dermatitis and other inflammatory skin pathologies, neuritis, encephalitis, encephalomyelitis and inflammatory pathologies affecting the central nervous system (multiple sclerosis) or peripheral nervous system, myositis, vasculitis, systemic lupus erythematosus, asthma, chronic obstructive lung disease, infectious diseases with inflammation symptoms, reactions of host rejection of grafts, conjunctivitis and inflammatory pathologies of the eye, ear, and mucous membranes.
